(19) Europäisches Patentamt
European Patent Office
Office européen des brevets

(11) Publication number: **0 012 988**
**B1**

(12) # EUROPEAN PATENT SPECIFICATION

(45) Date of publication of patent specification: **27.04.83**

(21) Application number: **79105257.4**

(22) Date of filing: **18.12.79**

(51) Int. Cl.³: **C 07 D 211/70, A 61 K 31/445**

(54) **Levorotatory or dextrorotatory enantiomers of 3-halocyproheptadines, process for their preparation and pharmaceutical composition thereof.**

(30) Priority: **02.01.79 US 323**

(43) Date of publication of application:
**09.07.80 Bulletin 80/14**

(45) Publication of the grant of the patent:
**27.04.83 Bulletin 83/17**

(84) Designated Contracting States:
**AT BE CH DE FR GB IT LU NL SE**

(56) References cited:
**US - A - 3 014 911**
**US - A - 4 031 222**
**US - A - 4 031 223**

**HELVETICA CHIMICA ACTA. vol. 48, no. 6, September 20, 1965 Basel CH EBNOTER A. et al. "No. 136. Atropisomerie in der Dibenzo (a,d) cyclohepten-Reihe", pages 1237—49**

**The file contains technical information submitted after the application was filed and not included in this specification**

(73) Proprietor: **MERCK & CO. INC.**
**126, East Lincoln Avenue P.O. Box 2000**
**Rahway New Jersey 07065 (US)**

(72) Inventor: **Remy, David C.**
**607 Jenkins Lane, M.R. 1**
**US-19454 North Wales, PA (US)**

(74) Representative: **Blum, Rudolf E. et al,**
**c/o E. Blum & Co Patentanwälte Vorderberg 11**
**CH-8044 Zürich (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

**EP 0 012 988 B1**

# Levorotatory or dextrorotatory enantiomers of 3-halocyproheptadines, process for their preparation and pharmaceutical composition thereof.

## Background of the Invention

This invention is concerned with new levorotatory or dextrorotatory enantiomers of 3-halocyproheptadines. The levorotatory 3-halocyproheptadines and the racemic mixtures are antipsychotic agents, and the invention is also concerned with pharmaceutical compositions containing an effective antipsychotic amount of the corresponding racemic or the levorotatory compound.

Racemic 3-bromocyproheptadine and 3-chlorocyproheptadine are disclosed in U.S. Patent 3,014,911 as members of a large group of cyproheptadine derivatives which possess antiserotonin and antihistamine activity. They are also disclosed in J. Med. Chem., *8*, 829 (1965).

The levorotatory enantiomer of 3-chlorocyproheptadine is disclosed by Ebnöther et al., in Helv. Chim. Acta, *48*(6), 1237—1249 (1965). The levorotatory enantiomer of 3-iodocyproheptadine is disclosed in U.S. Patent 4,031,223 and the levorotatory enantiomer of 1-cyclopropylmethyl-4-(3-iodo-5H-dibenzo[a,d]cyclohepten-5-ylidene)piperidine is disclosed in U.S. Patent 4,031,222.

There are provided certain novel compounds within the class of 3-halocyproheptadines and analogs thereof and a novel process for synthesizing these novel compounds.

## Detailed Description of the Invention

The novel compounds of this invention are the levorotatory and dextrorotatory enantiomers of the compounds having the following structural formula I:

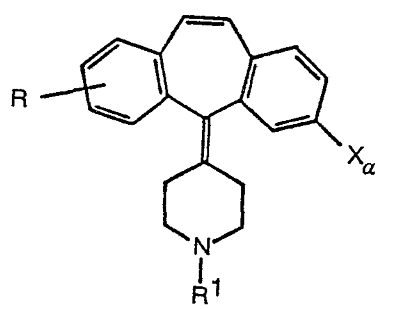

(I)

or pharmaceutically acceptable salts thereof, wherein $X_\alpha$ represents bromo or chloro; R represents hydrogen, $C_{1-3}$ alkyl, or fluoro; $R^1$ represents methyl or cyclopropylmethyl, when $X_\alpha$ is bromo; and $R^1$ represents cyclopropylmethyl when $X_\alpha$ is chloro.

As already mentioned above in the publication in Helv. Chim. Acta, *48* (6) there is disclosed on page 1238 the 3-chloro-cyproheptadiene, which compound corresponds to the above formula I, if $X_\alpha$ has the meaning of a chloro atom, however in said compound $R^1$ is a methyl group.

The compound disclosed in the U.S. Patent 4,031,223 as well is related to the compound of formula I, in said compounds however $R^1$ has to be a methyl group while $X_\alpha$ is either iodo or the group —$SCF_3$.

In the above mentioned U.S. Patent 4,031,222 there are furthermore described compounds corresponding to formula I, in which $R^1$ is the cyclopropylmethyl group, however in said compounds the substituent corresponding to $X_\alpha$ has to be iodo or the group —$SCF_3$.

Tests of comparison showed that the levorotatory enantiomer of the new compounds of formula I have a high antipsychotic activity and less side effects than the levorotatory enantiomers of the compounds described in the U.S. Patents 4,031,221 and 4,031,223.

A preferred embodiment of the novel compounds of formula I is that wherein R is hydrogen. An even more preferred embodiment of the novel compound is that, wherein R is hydrogen and X is bromo.

An especially preferred compound of formula I is the racemic or the levorotatory enantiomer or the dextrorotatory enantiomer of 1-methyl-4-(3-bromo-5H-dibenzo(a,d)cyclohepten-5-ylidene)piperidine and phamaceutically acceptable salts of said substances.

A further object of the present invention is an antipsychotic composition in unit dosage form comprising a pharmaceutical carrier and an effective antipsychotic amount of a racemic or levorotatory compound of formula I:

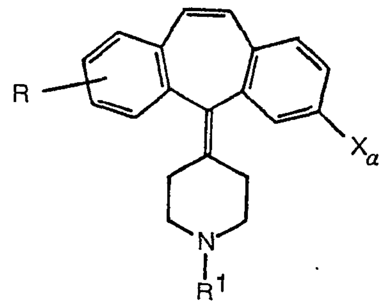

(I)

or pharmaceutically acceptable salts thereof, wherein

$X_\alpha$ is bromo or chloro;

R is hydrogen, fluoro or $C_{1-3}$ alkyl; and

$R^1$ is methyl or cyclopropylmethyl, when $X_\alpha$ is bromo, and

$R^1$ is cyclopropylmethyl, when $X_\alpha$ is chloro.

Preferred pharmaceutical compositions comprise compounds of formula I wherein R is hydrogen and X is bromo, and preferably the racemic or levorotatory enantiomer of 1-methyl-4-(3-bromo-5H-dibenzo(a,d)-cyclohepten-5-ylidene)-piperidine or pharmaceutically acceptable salts thereof.

The present invention furthermore concerns a process for the preparation of the levorotatory and dextrorotatory enantiomers of a compound of formula I:

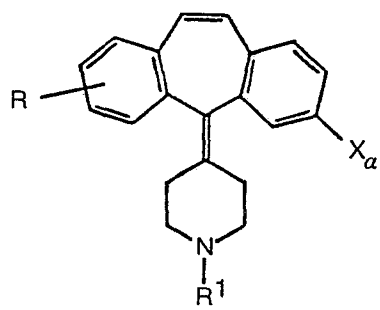

(I)

or pharmaceutically acceptable salts thereof, wherein

$X_\alpha$ is bromo or chloro,

R is hydrogen, fluoro or $C_{1-3}$ alkyl, and

$R^1$ is methyl or cyclopropylmethyl when $X_\alpha$ is bromo, and

$R^1$ is cyclopropylmethyl when $X_\alpha$ is chloro, which is characterized in that a compound of the structural formula II

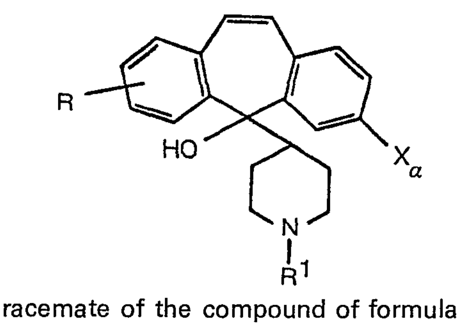

(II)

is dehydrated to form the racemate of the compound of formula I, followed by the resolution of the racemate.

The pharmaceutically acceptable salts of the novel compounds of this invention are acid addition salts formed from the novel compounds of formula I and an organic or inorganic acid recognized by the art as providing a pharmaceutically acceptable acid addition salt, such as hydrochloride, hydrobromide, dihydrogen phosphate, sulfate, pamoate, citrate, napsylate, pyruvate, isethionate, maleate or fumarate.

The salts are prepared by dissolving approximately equimolecular amounts of the free base compound and the desired acid in a solvent followed by crystallization of the salt product.

The novel process for the preparation of the compounds of this invention comprises dehydration of an R-substituted 1-$R^1$-4-(3-$X_\alpha$-5-hydroxy-5H-dibenzo[a,d]cyclohepten-5-yl)piperidine with a dehydrating agent such as trifluoroacetic acid/trifluoroacetic anhydride at reflux temperature, as described in J. Med. Chem., *8*, 829 (1965), to form a racemic mixture of the novel compounds of this

invention. The racemic mixture is then resolved by formation of diastereomeric salts with it and an optically active acid such as di-*p*-toluoyl-*d*-tartaric acid in a solvent such as ethanol followed by separation of the diastereomeric pair of salts such as by fractional crystallization followed by separate treatment of each salt with an alkali such as an alkali metal hydroxide, bicarbonate or carbonate, especially sodium bicarbonate or carbonate to liberate the free (+)- and (—)- enantiomers. The levorotatory isomer is further resolved via recrystallization from a solvent such as acetonitrile.

The optically enriched dextrorotatory compound obtained as described above can be racemized by heating a solution of it in an inert solvent until a sample fails to show optical activity. It is convenient to reflux a toluene solution for about 10—50 hours. In this manner, additional quantities of the racemic compound can be obtained from which additional levorotatory material can be isolated by the above described resolution.

The novel pharmaceutical compositions of this invention contain the racemic or levorotatory compounds of formula I or a pharmaceutically acceptable salt thereof and a pharmaceutical carrier.

The pharmaceutically acceptable salts contemplated for this purpose are the same salts discussed herein in connection with the group of novel compounds.

As pointed out by Ebnöther et al., Helv. Chim. Acta, *48*, 1237—1249 (1965) these compounds exist as levorotatory and dextrorotatory optical enantiomers. All of the antipsychotic activity resides in the levorotatory enantiomers, but the racemic mixtures of the levo- and dextrorotatory enantiomers, the mixture from which the levorotatory enantiomers are obtained are still potent antipsychotic agents and are useful in the novel pharmaceutical compositions of this invention. Thus there is contemplated for use in the novel pharmaceutical compositions:

(1) racemic mixtures of levo- and dextrorotatory enantiomers, herein after referred to as "racemic compounds"; and

(2) any mixtures optically enriched in the levorotatory sense or pure levorotatory enantiomers, hereinafter referred to as "levorotatory compounds".

The novel pharmaceutical compositions are used for the administration of an antipsychotically effective amount of one of the racemic or levorotatory compounds of formula I or a pharmaceutically acceptable salt thereof to a psychotic patient. The route of administration can be oral, rectal, intravenous, intramuscular, or subcutaneous. Doses of 0.1 to 20 mg./kg./day and preferably of 0.5 to 10 mg./kg./day of active ingredient are adequate, and if preferred, it can be administered in divided doses given two to four times daily.

It is to be noted that the precise unit dosage form and dosage level depend upon the case history of the individual being treated and, consequently, are left to the discretion of the therapist.

Pharmaceutical compositions comprising a compound of structural formula I as active ingredient may be in any art recognized form suitable for oral use, such as tablets, troches, lozenges, aqueous or oil suspensions, dispersible powders, or granules, emulsions, hard or soft capsules, syrups, or elixirs. For intravenous and intramuscular and subcutaneous use the pharmaceutical compositions may be in any art recognized form of a sterile injectable preparation such as a sterile aqueous or oleaginous solution or suspension. The amount of active ingredient incorporated in a unit dosage of the above described pharmaceutical compositions may be from 1 to 400 mg., and preferably from 5 to 250 mg.

Example 1

(—)-1-Methyl-4-(3-bromo-5H-dibenzo[a,d]cyclohepten-5-ylidene)piperidine

*Step A:* Preparation of 1-methyl-4-(3-bromo-5-hydroxy-5H-dibenzo[a,d]cyclohepten-5-yl)piperidine

To an ice cooled solution of 2.10 g. (0.0074 mol) of 3-bromo-5H-dibenzo[a,d]cyclohepten-5-one in 35 ml. of dry tetrahydrofuran is added dropwise 36 ml. of 0.41 *M* 1-methyl-4-piperidylmagnesium chloride. The solution is stirred for one hour and then the tetrahydrofuran is removed by evaporation on a rotary evaporator. The red, oily residue that remains is dissolved in benzene and water is added dropwise until a clear benzene supernatant and a gelatinous aqueous phase is obtained. The benzene phase is decanted and the gelatinous aqueous phase is extracted with four 50 ml. portions of hot benzene. The combined benzene phases are washed with water, dried over magnesium sulfate, filtered, and benzene is removed on a rotary evaporator. The residue is triturated with cold acetonitrile and collected by filtration to give 0.86 g. (40%) of 1-methyl-4-(3-bromo-5-hydroxy-5H-dibenzo[a,d]cyclohepten-5-yl)piperidine.

*Step B:* Preparation of (±)-1-methyl-4-(3-bromo-5H-dibenzo[a,d]cyclohepten-5-ylidene)piperidine.

A solution of 0.86 g. (0.003 mol) of 1-methyl-4-(3-bromo-5-hydroxy-5H-dibenzo[a,d]cyclohepten-5-yl)piperidine in 30 ml. of trifluoroacetic acid and 15 ml. of trifluoroacetic anhydride is stirred and refluxed for 16 hours. The solvents are removed by evaporation on a rotary evaporator. The residue is dissolved in chloroform, and this chloroform solution is washed with sodium hydroxide solution, water, dried over magnesium sulfate, and filtered. Evaporation of the chloroform from the filtrate gives 0.88 g. of a yellow oil. This oil is dissolved in a minimum amount of absolute ethanol, treated with ethanolic HCl, and is cooled. The white crystalline material that precipitates is collected by filtration and is recrystallized from acetonitrile to give 0.67 g. of (±)-1-methyl-4-(3-bromo-5H-dibenzo[a,d]cyclohepten-5-ylidene)piperidine hydrochloride.

*Step C:* Preparation of (—)-1-methyl-4-(3-bromo-5H-dibenzo[a,d]cyclohepten-5-ylidene)piperidine.

To a solution of 12.42 g. (0.0339 mol) of (±)-1-methyl-4-(3-bromo-5H-dibenzo[a,d]cyclohepten-5-ylidene piperidine in 250 ml of hot ethanol is added 13.11 g. (0.0339 mol) of di-p-toluoyl-*d*-tartaric acid dissolved in 50 ml of warm ethanol. The solution is stirred and allowed to cool to room temperature. The salt that crystallizes is removed by filtration and is recrystallized from ethanol six times to afford 2.62 g. of material having a constant rotation: $[\alpha]^{25}_{589}$ —111°, $[\alpha]^{25}_{578}$ —116°, $[\alpha]^{25}_{546}$ —137°, $[\alpha]^{25}_{436}$ —306° (c, 0.531, pyridine). This salt is converted to the free base with saturated sodium bicarbonate solution and extracting it into ether. The ether phase is washed with water, dried over magnesium sulfate, filtered, and the ether is removed. Recrystallization from acetonitrile gives (—)-1-methyl-4-(3-bromo-5H-dibenzo[a,d]cyclohepten-5-ylidene)piperidine as TLC homogeneous (fl. alumina/$CHCl_3$), sparkling white prisms, m.p. 189—190°; $[\alpha]^{25}_{589}$ —100°, $[\alpha]^{25}_{578}$ —106°, $[\alpha]^{25}_{546}$ —127°, $[\alpha]^{25}_{436}$ —304° (c, 0.731, $CHCl_3$).

Anal. Calcd. for $C_{21}H_{20}BrN$: C,68.86; H,5.50; Br,21.82; N,3.82.
Found: C,68.97; H,5.58; Br,21.62; N,3.39.

Example 2

(+)-1-Methyl-4-(3-bromo-5H-dibenzo[a,d]cyclohepten-5-ylidene)piperidine

Starting with 5.76 g (0.0157 mol) of (±)-1-methyl-4-(3-bromo-5H-dibenzo[a,d]cyclohepten-5-ylidene)piperidine toluoyl-*l*-tartaric acid monohydrate in 25 ml of ethanol and using the procedure as described above, 1.60 g of crystalline salt is obtained $[\alpha]^{25}_{589}$ +110°; $[\alpha]^{25}_{578}$ +116°; $[\alpha]^{25}_{546}$ +137°; $[\alpha]^{25}_{436}$ +302° (c, 0.403, pyridine). Conversion to the free base and crystallization from acetonitrile gives (+)-1-methyl-4-(3-bromo-5H-dibenzo[a,d]cyclohepten-5-ylidene)piperidine, m.p. 189—191°C; $[\alpha]^{25}_{589}$ +100°, $[\alpha]^{25}_{578}$ +107, $[\alpha]^{25}_{546}$ +127, $[\alpha]^{25}_{436}$ +307° (c, 0.651, $CHCl_3$).

Employing the procedures substantially as described in Examples 1 and 2 but substituting for the 3-bromo-5H-dibenzo[a,d]cyclohepten-5-one and/or the 1-methyl-4-piperidylmagnesium chloride used in Example 1, Step A similar relative amounts of the 3-$X_\alpha$-7-R-5H-dibenzo[a,d]-cyclohepten-5-ones, and 1-$R^1$-4-piperidylmagnesium chloride, described in Table I, there are produced the (—)- and (+)-enantiomers of 1-$R^1$-4-(3-$X_\alpha$-7-R-5H-dibenzo[a,d]cyclohepten-5-ylidene)piperidine, also described in Table I in accordance with the following reaction scheme:

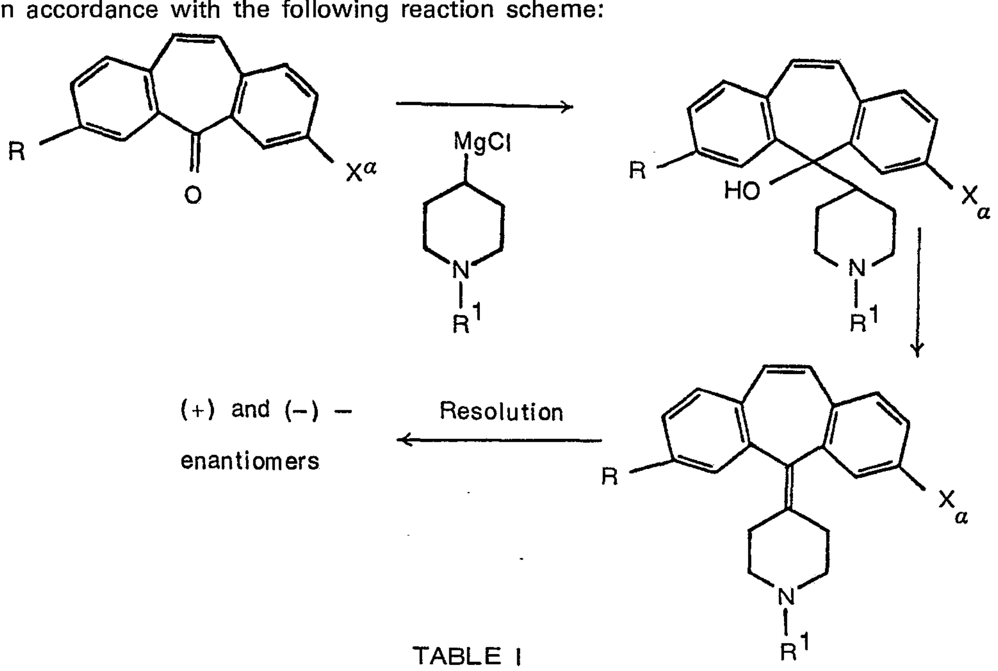


TABLE I

| R | $R^1$ | $X_\alpha$ |
|---|---|---|
| —F | $-CH_3$ | —Br |
| $-CH_3$ | $-CH_3$ | —Br |
| —H | $-CH_2-$◁ | —Br |
| —F | $-CH_2-$◁ | —Br |
| $-CH_3$ | $-CH_2-$◁ | —Br |
| H | $-CH_2-$◁ | —Cl |
| —F | $-CH_2-$◁ | —Cl |
| $-CH_3$ | $-CH_2-$◁ | —Cl |

Example 3

Pharmaceutical Compositions

A typical tablet containing 100 mg. of (—)-1-methyl-4-(3-bromo-5H-dibenzo[a,d]cyclohepten-5-ylidene)piperidine per tablet is prepared by mixing together with the active ingredient calcium phosphate, lactose and starch in the amounts shown in the table below. After these ingredients are thoroughly mixed, the appropriate amount of magnesium stearate is added and the dry mixture is then compressed into tablets.

Tablet Formula

| Ingredient | Mg. per Tablet |
|---|---|
| (—)-1-methyl-4-(3-bromo-5H-dibenzo[a,d]-cyclohepten-5-ylidene)piperidine | 100 mg. |
| Calcium phosphate | 52 mg. |
| Lactose | 60 mg. |
| Starch | 10 mg. |
| Magnesium stearate | 1 mg. |

Similarly tablets containing the other racemic or levorotatory compounds active in the novel compositions of this invention are prepared by substituting for the 100 mg ($2.7 \times 10^{-4}$ mole) of (—)-1-methyl-4-(3-bromo-5H-dibenzo[a,d]cyclohepten-5-ylidene)piperidine a comparable molecular amount of any of the racemic or levorotatory compounds with structural formula I or a pharmaceutically acceptable salt thereof.

Pharmacology

Antagonism of (+)-Amphetamine

*Methods:*

Mice ($CF_1$ females weighing about 20 g.) were injected i.p. with the test compounds two hours and five minutes prior to a subcutaneous administration of (+)-amphetamine, 10 mg./kg. Forty-five minutes after giving (+)-amphetamine, the mice were observed for the presence or absence of excitement and locomotor stimulation elicited by (+)-amphetamine.

*Results:*

At a dose of 30 mg./kg. of (—)-1-methyl-4-(3-bromo-5H-dibenzo[a,d]cyclohepten-5-ylidene)piperidine (L 636,524), 80% of the mice failed to exhibit the typical signs normally elicited by (+)-amphetamine. A reference neuroleptic, chlorpromazine, also antagonized the action of (+)-amphetamine (Table 1).

Table 1.
Antagonism of (+)-amphetamine-induced excitement and hyperactivity.

| Treatment[a] (mg./kg. i.p.) | | # Protected[b] / # Tested |
|---|---|---|
| L—636,524—OOY—01 | (6) | 0/5 |
| " | (30) | 4/5 |
| " | (150) | 5/5 |
| Chlorpromazine | (6) | 5/5 |
| " | (30) | 5/5 |
| " | (150) | |

[a] Two hours and five minutes before (+)-amphetamine (10 mg./kg. s.c.).
[b] Mice were observed 45 minutes after (+)-amphetamine.

**Claims**

1. A levorotatory or dextrorotatory enantiomer of a compound of structural formula I:

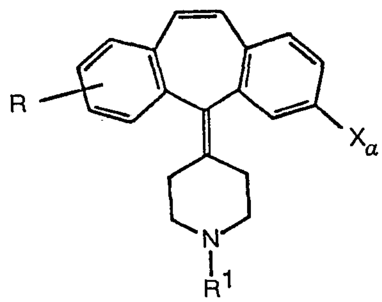

(I)

or pharmaceutically acceptable salts thereof, wherein $X_\alpha$ is bromo or chloro; R is hydrogen, fluoro or $C_{1-3}$ alkyl; and $R^1$ is methyl or cyclopropylmethyl when $X_\alpha$ is bromo; and $R^1$ is cyclopropylmethyl when $X_\alpha$ is chloro.

2. The compound of Claim 1 which is the levorotatory enantiomer of 1-methyl-4-(3-bromo-5H-dibenzo[a,d]cyclohepten-5-ylidene)piperidine or pharmaceutically acceptable salts thereof.

3. The compound of Claim 1, which is the dextrorotatory enantiomer of 1-methyl-4-(3-bromo-5H-dibenzo[a,d]cyclohepten-5-ylidene)piperidine or pharmaceutically acceptable salts thereof.

4. An antipsychotic composition in unit dosage form comprising a pharmaceutical carrier and an effective antipsychotic amount of a racemic or levorotatory compound of formula I:

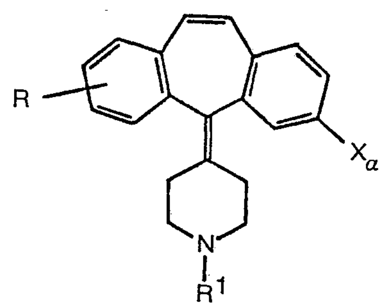

(I)

or pharmaceutically acceptable salts thereof, wherein

$X_\alpha$ is bromo or chloro;

R is hydrogen, fluoro or $C_{1-3}$ alkyl; and

$R^1$ is methyl or cyclopropylmethyl, when $X_\alpha$ is bromo, and

$R^1$ is cyclopropylmethyl, when $X_\alpha$ is chloro.

5. The pharmaceutical composition of claim 4, wherein R is hydrogen and $X_\alpha$ is bromo.

6. The pharmaceutical composition of claim 5, wherein the racemic or levorotatory compound is 1-methyl-4-(3-bromo-5H-dibenzo(a,d)-cyclohepten-5-ylidene)piperidine or pharmaceutically acceptable salts thereof.

7. A process for preparation of the levorotatory and dextrorotatory enantiomers of a compound of formula I:

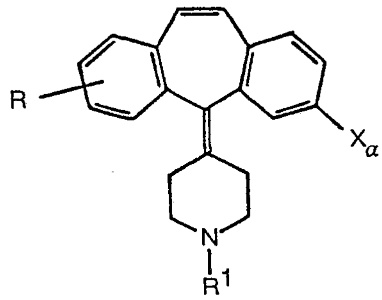

(I)

or pharmaceutically acceptable salts thereof, wherein

$X_\alpha$ is bromo or chloro,

R is hydrogen, fluoro or $C_{1-3}$ alkyl, and

$R^1$ is methyl or cyclopropylmethyl when $X_\alpha$ is bromo, and

$R^1$ is cyclopropylmethyl when $X_\alpha$ is chloro, characterized in that a compound of the structural formula II:

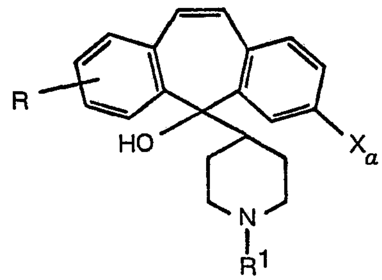

(II)

is dehydrated to form the racemate of the compound of formula I, followed by the resolution of the racemate.

8. The process of claim 7, wherein the levorotatory enantiomer of 1-methyl-4-(3-bromo-5H-dibenzo(a,d)cyclohepten-5-ylidene)piperidine or pharmaceutically acceptable salts thereof are prepared.

**Revendications**

1. Un énantiomère lévogyre ou dextrogyre d'un composé de formule développée (I):

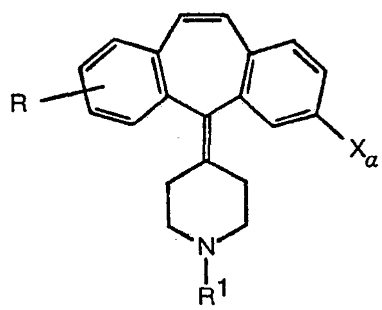

(I)

ou un sel acceptable en pharmacie correspondant, où

$X_\alpha$ est un bromo ou un chloro;

R est un hydrogène, un fluoro ou un alkyle en $C_{1-3}$; et

$R^1$ est un méthyle ou un cyclopropylméthyle lorsque $X_\alpha$ est un bromo; et

$R^1$ est un cyclopropylméthyle lorsque $X_\alpha$ est un chloro.

2. Le composé de la revendication 1 qui est l'énantiomère lévogyre de la 1-méthyl-4-(3-bromo-5H-dibenzo[a,d]cycloheptène-5-ylidène)pipéridine ou un sel acceptable en pharmacie correspondant.

3. Le composé de la revendication 1 qui est l'énantiomère dextrogyre de la 1-méthyl-4-(3-bromo-5-dibenzo[a,d]cycloheptène-5-ylidène)pipéridine ou un sel acceptable en pharmacie correspondant.

4. Composition antipsychotique sous une forme d'administration unitaire comprenant un véhicule pharmaceutique et une quantité antipsychotique efficace d'un composé racémique ou lévogyre de formule (I):

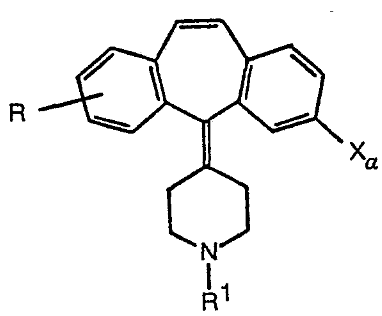

(I)

ou de sels acceptables en pharmacie correspondants, où

$X_\alpha$ est un bromo ou un chloro;

R est un hydrogène, un fluoro ou un alkyle en $C_{1-3}$; et

$R^1$ est un méthyle ou un cyclopropylméthyle lorsque $X_\alpha$ est un bromo; et

$R^1$ est un cyclopropylméthyle lorsque $X_\alpha$ est un chloro.

5. La composition pharmaceutique de la revendication 4 où R est un hydrogène et $X_\alpha$ est un bromo.

6. La composition pharmaceutique de la revendication 5 où le composé racémique ou lévogyre est la 1-méthyl-4-(3-bromo-5H-dibenzo[a,d]cycloheptène-5-ylidène)pipéridine ou un sel acceptable en pharmacie correspondant.

7. Procédé pour la préparation des énantiomères lévogyres et dextrogyres d'un composé de formule (I):

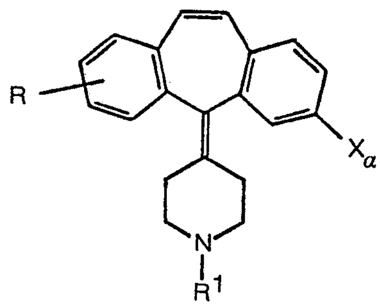

(I)

ou des sels acceptables en pharmacie correspondants, où

$X_\alpha$ est un bromo ou un chloro;

R est un hydrogène, un fluoro ou un alkyle en $C_{1-3}$

$R^1$ est un méthyle ou un cyclopropylméthyle lorsque $X_\alpha$ est un bromo; et

$R^1$ est un cyclopropylméthyle lorsque $X_\alpha$ est un chloro,

caractérisé en ce qu'on déshydrate un composé de formule développée (II):

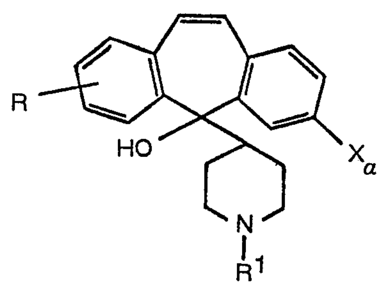

pour former le racémate du composé de formule (I); puis on dédouble le racémate.

8. Le procédé de la revendication 7, dans lequel on prépare l'énantiomère lévogyre de la 1-méthyl-4-(3-bromo-5H-dibenzo[a,d]cycloheptène-5-ylidène)pipéridine ou les sels acceptables en pharmacie correspondants.

**Patentansprüche**

1. Ein linksdrehendes oder rechtsdrehendes Enantiomeres einer Verbindung mit der Strukturformel I

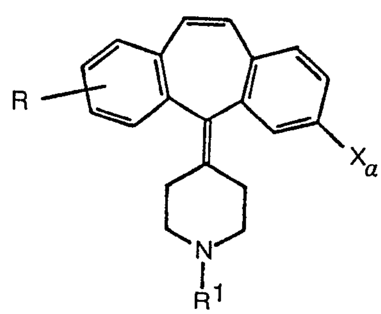

oder ein pharmazeutisch brauchbares Salz davon, worin $X_\alpha$ Brom oder Chlor ist; R Wasserstoff, Fluor oder $C_{1-3}$-Alkyl ist; und $R^1$ Methyl oder Cyclopropylmethyl ist, wenn $X_\alpha$ Brom ist; und $R^1$ Cyclopropylmethyl ist, wenn $X_\alpha$ Chlor ist.

2. Verbindung nach Anspruch 1, bei der es sich um das linksdrehende Enantiomere von 1-Methyl-4-(3-brom-5H-dibenzo[a,d]cyclohepten-5-yliden)piperidin handelt oder pharmazeutisch brauchbare Salze davon.

3. Verbindung nach Anspruch 1, bei der es sich um das rechtsdrehende Enantiomere von 1-Methyl-4-(3-brom-5H-dibenzo[a,d]cyclohepten-5-yliden)piperidin handelt oder pharmazeutisch brauchbare Salze davon.

4. Antipsychotische Zusammensetzung in Dosiseinheitsform, enthaltend einen pharmazeutischen Träger und eine wirksame antipsychotische Menge einer racemischen oder linksdrehenden Verbindung der Formel I

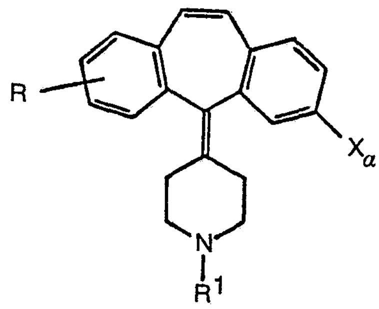

(I)

oder pharmazeutisch brauchbare Salze davon, worin

$X_\alpha$ Brom oder Chlor ist;
R Wasserstoff, Fluor oder $C_{1-3}$-Alkyl ist; und
$R^1$ Methyl oder Cyclopropylmethyl ist, wenn $X_\alpha$ Brom ist und
$R^1$ Cyclopropylmethyl ist, wenn $X_\alpha$ Chlor ist.

5. Pharmazeutische Zusammensetzung nach Anspruch 4, worin R Wasserstoff ist und X Brom ist.

6. Pharmazeutische Zusammensetzung nach Anspruch 5, in der die racemische oder linksdrehende Verbindung 1-Methyl-4-(3-brom-5H-dibenzo(a,d)-cyclohepten-5-yliden)-piperidin oder ein pharmazeutische brauchbares Salz davon ist.

7. Verfahren zur Herstellung der linksdrehenden und rechtsdrehenden Enantiomeren einer Verbindung der Formel I:

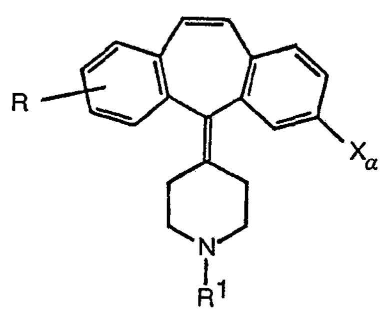

oder der pharmazeutisch brauchbaren Salze davon, worin

$X_\alpha$ Brom oder Chlor ist,
R Wasserstoff, Fluor oder $C_{1-3}$-Alkyl ist, und
$R^1$ Methyl oder Cyclopropylmethyl ist, wenn $X_\alpha$ Brom ist und
$R^1$ Cyclopropylmethyl ist, wenn $X_\alpha$ Chlor ist,

dadurch gekennzeichnet, dass eine Verbindung der Strukturformel II

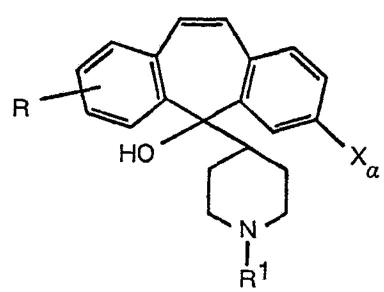

(II)

dehydratisiert wird unter Bildung des Racemats der Verbindung der Formel I, worauf das Racemat gespalten wird.

8. Verfahren nach Anspruch 7, bei dem das linksdrehende Enantiomere von 1-Methyl-4-(3-brom-5H-dibenzo(a,d)-cyclohepten-5-yliden)piperidin oder pharmazeutisch brauchbare Salze davon hergestellt werden.